# EUROPEAN PATENT APPLICATION

(11) **EP 1 721 617 A2**
(43) Date of publication of application: **15.11.2006**
(21) Application number: 06009543.7
(22) Date of filing: 22.07.1998
(51) Int. Cl.: A61K 38/29, A61P 19/08

(54) **Dental therapeutics containing parathyroid hormone**

(30) Priority: 22.07.1997 JP 19531597
(62) Divisional of application: 98933885.0
(71) Applicant: CHUGAI SEIYAKU KABUSHIKI KAISHA, Tokyo, 115-8543 (JP)
(72) Inventor: Horiuchi, Noboru, Koriyama-shi Fukushima 963-8051 (JP); Kawane, Tetsuya, Koriyama-shi Fukushima 963-8021 (JP)
(74) Representative: Vossius & Partner

(57) **Abstract**

Dental therapeutics containing (1) parathyroid hormone (PTH) or one or more PTH derivatives or (2) parathyroid hormone (PTH) or one or more PTH derivatives and estrogen as an active ingredient or active ingredients have various actions such as increasing the bone density of the jaw alveolar bone portion, particularly the one in the upper jaw or the lower jaw, preventing or treating the weakening of the jaw alveolar bone portion, particularly the one in the upper jaw or the lower jaw, promoting the regeneration of the jaw alveolar bone portion, particularly the one in the upper jaw or the lower jaw, and increasing the bone densities of the whole mandibular bone and the mandibular molar portion after tooth removal; hence, they are clinically effective.

## Description

### TECHNICAL FIELD

This invention relates to dental therapeutics containing (1) parathyroid hormone (PTH) or PTH derivatives or (2) parathyroid hormone (PTH) or PTH derivatives and estrogen as an active ingredient or active ingredients, for example, those for increasing the bone density of the jaw alveolar bone portion, particularly the one in the upper jaw or the lower jaw, preventives or therapeutics of the weakening of the jaw alveolar bone portion, particularly the one in the upper jaw or the lower jaw, promoters of the regeneration of the jaw alveolar bone, particularly the one in the upper jaw or the lower jaw, and dental therapeutics to be applied after tooth removal or upon tooth loss.

### BACKGROUND ART

Parathyroid hormone (PTH) is known as an important hormone in bone metabolism. A multitude of reports have so far been published to describe the action of PTH on the bone.

It is well known that parathyroid hormone (PTH) is one of the essential systemic factors in bone remodeling. Intermittent injections of PTH *in vivo* bring about an increase in the bone mass of ovariectomized (OVX) rats (Hock et al.; 1988; Liu et al., 1991; Ibbotson, 1992) or healthy rats (Hock and Gera, 1992; Dobnig, 1995). Therefore, stimulation of bone formation is regarded as one of the physiological roles of the pulsating secretion of PTH *in* vivo. On the other hand, bone morphometry on parathyroidectomized rats (Kitazawa et al., 1991) or normal dogs (Malluche et al., 1982) submitted to continuous injection of PTH showed simultaneous increases in bone formation and bone resorption but not a net increase in bone mass. According to the data obtained by various in vivo studies, osteoclast formation (Takahashi et al., 1988; Kurihara et al., 1991) and the growth of osteoblasts (Somjen et al., 1990) are both stimulated by PTH. It has also been reported that since the periodontal tissue is subject to systemic influences from PTH administration, the alkali phosphatase reaction of the periodontium and the distribution of osteoclasts present with different pictures than those found in the control group (Toshio Ideguchi, Journal of the Society of Orthodontics of Japan, Vol. 28, No. 1, 1969, pp. 1 - 7).

However, not a single practical application of PTH at dental clinical sites has so far been identified.

In terms of age, patients with osteoporosis overlap people who have to be fitted with plate denture or implants, so osteoporosis is one of the diseases that receive particular attention in dentistry. For diagnosis of osteoporosis, bone density is measured by dual energy X-ray absorptiometry (DXA) and lowered bone densities, typically in the femur of ovariectomized (OVX) rats have been reported (Shen V., Birchman R., Xu R. et al., J. Clin. Invest. 96:2331-2338, 1995; Mitlak BH., Burdette-Miller P., Schoenfeld D. et al., J. Bone and Mineral Research 11:430-439, 1996). However, the effects of OVX on bone density have not been fully unravelled in the jaw bones. In addition, it is not clear what effects are caused on the jaw bones by administering estrogens as preventives or therapeutics of osteoporosis or parathyroid hormone (PTH) which is a physiologically active substance with a bone formation promoting action that has recently gained increasing attention.

The purpose of the present invention is to provide dental therapeutics that are practically feasible and effective in dental clinical applications.

### DISCLOSURE OF INVENTION

As the result of their intensive studies, the present inventors found that administration of (1) parathyroid hormone (PTH) or one or more PTH derivatives or (2) PTH or one or more PTH derivatives and estrogen elevated the bone density of the jaw alveolar bone portion, particularly the one in the upper jaw or the lower jaw, could prevent or treat the weakening of the jaw alveolar bone portion, particularly the one in the upper jaw or the lower jaw, was applicable as a promoter of bone regeneration after placement of implants and elevated the mandibular bone density after tooth removal. From these results, (1) or (2) was found to be applicable as a dental therapeutic. The present invention has been accomplished on the basis of these findings.

Thus, the present invention relates to a dental therapeutic containing (1) parathyroid hormone (PTH) or one or more PTH derivatives or (2) PTH or one or more PTH derivatives and estrogen as an active ingredient or active ingredients. The present invention also relates to an enhancer of the bone density of the jaw alveolar bone portion, particularly the one in the upper jaw or the lower jaw, that contains (1) parathyroid hormone (PTH) or one or more PTH derivatives or (2) PTH or one or more PTH derivatives and estrogen as an active ingredient or active ingredients. The present invention further relates to a preventive or therapeutic of the weakening of the jaw alveolar bone portion, particularly the one in the upper jaw or the lower jaw, that contains (1) parathyroid hormone (PTH) or one or more PTH derivatives or (2) PTH or one or more PTH derivatives and estrogen as an active ingredient or active ingredients. Further in addition, the present invention relates to a promoter of the regeneration of the jaw alveolar bone portion, particularly the one in the upper jaw or the lower jaw, that contains (1) parathyroid hormone (PTH) or one or more PTH derivatives or (2) PTH or one or more PTH derivatives and estrogen as an active ingredient or active ingredients. The present invention also relates to a dental therapeutic to be applied after tooth removal or upon tooth loss that contains (1) parathyroid hormone (PTH) or one or more PTH derivatives or (2) PTH or one or more PTH derivatives and estrogen as an active ingredient or active ingredients. The present invention further relates to a dental composition containing (1) parathyroid hormone (PTH) or one or more PTH derivatives or (2) PTH or one or more PTH derivatives and estrogen as an active ingredient or active ingredients. The present invention still further relates to a PTH non-invasive preparation characterized by sustained administration of (1) an effective amount of parathyroid hormone (PTH) or one or more PTH derivatives or (2) an effective amount of parathyroid hormone (PTH) or one or more PTH derivatives and an effective amount of estrogen.

The dental therapeutics of the invention are typically used as an enhancer of the bone density of the jaw alveolar bone portion, a preventive or therapeutic of the weakening of the jaw alveolar bone portion, a promoter of the regeneration of the jaw alveolar bone portion or as an enhancer of the bone density of the mandible or the like after tooth removal or upon tooth loss. The terms "dental therapeutics", "an enhancer of the bone density of the jaw alveolar bone portion", "a preventive or therapeutic of the weakening of the jaw alveolar bone portion" and "a promoter of the regeneration of the jaw alveolar bone portion" mean those pharmaceutical agents used to prevent or treat bone abnormalities in the teeth, upper jaw and/or lower jaw. The dental therapeutics of the invention have an outstanding periodontal tissue regeneration promoting action and are useful not only in the regeneration of the periodontal tissues (the cementum, periodontium and alveolar bone) lost with the progress of periodontitis but also in the treatments of associated diseases such as repair of the osseous tissue after tooth removal and cyst or oral cancer extraction, reconstruction of a resorbed or atrophied alveolar bone, promoted fixing of implants and regeneration of the dentinum lost in dental caries; they are therefore used in the treatment of various periodontal diseases. In addition, the dental therapeutics of the invention promote the increase in bone minerals in the lower jaw bone after tooth removal, thereby increasing the mandibular bone density; hence, clinically, more noticeable efficacy is anticipated in patients suffering from tooth loss, for example, patients, in particular, elderly ones who have lost antagonistic teeth or patients with edentulous jaws.

### BRIEF DESCRIPTION OF DRAWINGS

Fig. 1 is a graph showing the differences in the bone density of the jaw molar portion (alveolar bone) depending on whether OVX was practiced, whether human PTH (1-84) was administered and whether 17β-estradiol was administered;
Fig. 2 is a graph showing the differences in the bone density of the jaw ramal portion depending on whether OVX was practiced, whether human PTH (1-84) was administered and whether 17β-estradiol was administered;
Fig. 3 is a graph showing the differences in the bone density of the whole mandibular bone depending on whether OVX was practiced, whether tooth removal was effected and whether human PTH (1-84) was administered; and
Fig. 4 is a graph showing the differences in the bone density of the mandibular molar portion depending on whether OVX was practiced, whether tooth removal was effected and whether human PTH (1-84) was administered.

### BEST MODE FOR CARRYING OUT THE INVENTION

The parathyroid hormone (PTH) to be used in the invention encompasses the natural type of PTH, recombinant PTH produced by genetic engineering techniques and chemically synthesized PTH. Preferred examples of said PTH include human PTH consisting of 84 amino acid residues (human PTH (1-84)), particularly recombinant human PTH (1-84) produced by genetic engineering techniques. The PTH derivatives include partial peptides of said PTH, peptides prepared by replacing a portion of the constituent amino acids in PTH per se or partial peptides thereof with other amino acids, peptides prepared by deleting a portion of the constituent amino acids in PTH per se or partial peptides thereof and peptides prepared by adding one or more amino acids to PTH per se or partial peptides thereof; all other peptides having similar activities are also contemplated. Exemplary partial peptides of PTH include human PTH (1-34), human PTH (1-64), human PTH (35-84) and bovine PTH (1-34). PTH (1-34) is a partial peptide of PTH comprising 34 amino acids starting at the N terminus of PTH and ending at the 34th amino acid. An example of the preferred partial peptide of PTH is human PTH comprising 34 amino acid residues (human PTH (1-34)), particularly recombinant human PTH (1-34) prepared by genetic engineering techniques.

Preferred examples of amino acid replacements include the replacement of the constituent amino acid in position 8 with leucine or norleucine, the replacement of the constituent amino acid in position 18 with leucine or norleucine, and the replacement of the constituent amino acid in position 34 with tyrosine.

In the present invention, parathyroid hormone (PTH) or PTH derivatives are used as dental therapeutics, or including an enhancer of the bone density of the jaw alveolar bone portion, particularly the one in the upper jaw or the lower jaw, a preventive or therapeutic of the weakening of the jaw alveolar bone portion, particularly the one in the upper jaw or the lower jaw, a promoter of the regeneration of the jaw alveolar bone portion, particularly the one in the upper jaw or the lower jaw and an enhancer of the bone density of the mandible or the like to be applied after tooth removal or upon tooth loss, or as dental compositions and PTH non-invasive preparations. Preferred examples of such parathyroid hormone (PTH) or PTH derivatives include human PTH (1-84), human PTH (1-34), human PTH (1-38), human PTH (1-37), and human PTH (1-34)-NH₂; more preferred examples include human PTH (1-84) and human PTH (1-34), and the most preferred example is human PTH (1-34).

In the present invention, parathyroid hormone (PTH) or PTH derivatives are used as dental therapeutics, or dental therapeutics including an enhancer of the bone density of the jaw alveolar bone portion, particularly the one in the upper jaw or the lower jaw, a preventive or therapeutic of the weakening of the jaw alveolar bone portion, particularly the one in the upper jaw or the lower jaw, a promoter of the regeneration of the jaw alveolar bone portion, particularly the one in the upper jaw or the lower jaw and an enhancer of the bone density of the mandible or the like to be applied after tooth removal or upon tooth loss. The parathyroid hormone (PTH) or PTH derivatives need not always be 100% pure but may be substantially pure. The term "substantially pure" as used in the invention means that the PTH or derivatives thereof should at least be purified to show a single peak in HPLC, preferably identified to be a single entity by its combination with other techniques such as SDS-PAGE and capillary electrophoresis. Such PTHs can be prepared and identified by the method described in Unexamined Published Japanese Patent Application (kokai) No. 87897/1994, or the methods described in Japanese Patent Domestic Announcement (kohyo) No. 505259/1992 and J. Biol. Chem., 265, 15854 (1990) or improvements thereof.

In the present invention, estrogen may be used together with parathyroid hormone (PTH) or PTH derivatives and the term "estrogen' means both natural estrogens (e.g., estrone, estradiol and estriol) and synthetic substances having an estrual action (e.g., stilbestrol). Preferred estrogens include 17β-estradiol (estra-1,3,5(10)-trien-3,17β-diol), estradiol benzoate (estra-1,3,5(10)-trien-3,17β-diol-3-monobenzoate), estradiol dipropionate (estra-1,3,5(10)-trien-3,17β-diol dipropionate), estradiol valerate (estra-1,3,5(10)-trien-3,17β-diol-17β-valerate), ethinyl estradiol (17α-ethynyl-1,3,5(10)-estratrien-3,17-diol), mestranol (ethynylestradiol-3-methyl-ether), estriol (estra-1,3,5(10)-trien-3,16α,17β-triol), estriol propionate (estra-1,3,5(10)-trien-3,16α,17β-triol tripropionate), estriol benzoacetate (estra-1,3,5(10)-trien-3,16α,17β-triol-3-benzoate-16,17-diacetate), and so forth. If estrogen is to be used together with PTH or PTH derivatives, one or more estrogens may be used either as pure compounds or in the form of a mixture.

The pharmaceutical agents of the invention may take on various dosage forms; in addition to injections (e.g., liquids and lyophilized preparations) that are produced by conventional pharmaceutical formulation procedures for peptides, they may be formulated in dosage forms that can allow for localized and long-standing efficacy, as exemplified by incorporation into microcapsules or impregnation in sheets of gel. When formulating, pharmaceutically acceptable auxiliary components may be added. With a view to increasing the half-life in blood, preparations modified with polyethylene glycol may be provided. A preferred example is non-invasive preparations.

Exemplary auxiliary components include base materials, stabilizers, antiseptics, preservatives, emulsions, suspending agents, solubilizers, solvent promoters, lubricants, flavoring agents, coloring agents, fragrances soothing agents, vehicles, binders, viscous agents, buffers and so forth; specific examples include calcium carbonate, lactose, sucrose, sobitol, mannitol, starch, amylopectin, cellulose derivatives, gelatin, cacao butter, water for injections, aqueous sodium chloride, Ringer's solution, glucose solution, human serum albumin, and so forth.

When preparing the pharmaceutical agents of the invention using the above-mentioned auxiliary components, they may be appropriately selected and used as described in, for example, A List of Additives for Medical Products (published by Committee on Medical Laws and Regulations, Foundation Association of Medical Product Industry, Tokyo and Committee on Medical Laws and Regulations, Foundation Association of Medical Product Industry, Osaka). The amounts in which the auxiliary components are to be used may be selected from pharmaceutically acceptable ranges as appropriate for dosage form and other factors.

The pharmaceutical agents of the invention may be administered either topically or systemically. If specific teeth (e.g. anterior teeth) are to be treated, topical administration is more advantageous. For topical administration, sustained application to a particular site is desirable. A preferred method for sustained application to a particular site is by either injecting PTH or a mixture of PTH and estrogen to the particular site or allowing them to be absorbed from the surface of a mucous membrane in a sustained manner, with a slow-release base material being used in either case. Specific examples of the slow-release base material, if it is to be used in direct submucous or subperiosteal injections, include (1) collagen pellets, (2) polylactic acid base, (3) hydroxyapatite cement, (4) alginic acid gel and so forth. Absorption from the surface of the mucous membrane can also be realized by an external applicator. The major advantage of systemic administration is that if an effective method is used, PTH or a mixture of PTH and estrogen can be administered without causing invasion. Preferred methods of systemic administration include systemic administration by subcutaneous, intravenous, intranasal and transpulmonary routes.

The administration period is in principle one of the following periods and determined etiolotically by the discretion of clinical doctors: the time required for the bone density of the jaw alveolar bone portion, particularly the one in the upper jaw or the lower jaw, to reach the necessary rate of increase; the time required for preventing or treating the weakening of the jaw alveolar bone portion, particularly the one in the upper jaw or the lower jaw; the time required for regenerating the jaw alveolar bone portion, particularly the one in the upper jaw or the lower jaw; the time required for the bone density of the whole mandibular bone or the mandibular molar portion to reach the necessary rate of increase after tooth removal or upon tooth loss. In terms of frequency, administration can be made at various intervals ranging from once a month to daily, preferably from about once in two weeks to five times a week or daily. Sustained administration is particularly preferred.

The dose of administration of PTH in the invention varies with the disease indicated for, pathology, severity of disease and other factors; for systemic administration, the dose ranges from about 1 µg to 1000 µg per kilogram of body weight, preferably from 5 µg to 200 µg per kilogram of body weight. The dose of administration of estrogen also varies with the disease indicated for, pathology, severity of the disease and other factors; for systemic administration, the dose ranges from 1 µg to 50 µg, preferably from about 2 µg to 20 µg per kilogram of body weight.

### Examples

The following examples are provided for the purpose of further illustrating the present invention but are in no way to be taken as limiting.

### Example 1

### (Method)

Forty female SD rats were used. At the age of 20 weeks after birth, 35 of the animals were subjected to OVX (ovariectomy) and the remaining five were subjected to Sham operation. Starting at the age of 30 weeks when bone resorption progressed fully, animals of the OVX group were subcutaneously administered with hormones for 10 weeks on a four-times-a-week basis. Five of the group administered with human PTH (1-84) were administered a dose of 10 µg/kg body weight, another five a dose of 40 µg/kg body weight and the last five a dose of 150 µg/kg body weight. The group administered with 17β-estradiol as estrogen consisted of 5 rats, which were administered a dose of 10 µg/kg body weight. Ten rats were administered with both hormones, 17β-estradiol at a dose of 10 µg/kg body weight and human PTH (1-84) at different doses of 10 µg/kg body weight (in 5 rats) and 150 µg/kg body weight (in the other five). After the hormone administration and at the age of 40 weeks after birth, the mandibular bone, femur and lumbar vertebrae were extracted and measured for bone density and bone mineral content by DXA (Aloca). Measurement on the mandibular bone was conduced at two sites, the alveolar bone portion including the posterior teeth and the ramal portion.

### (Results)

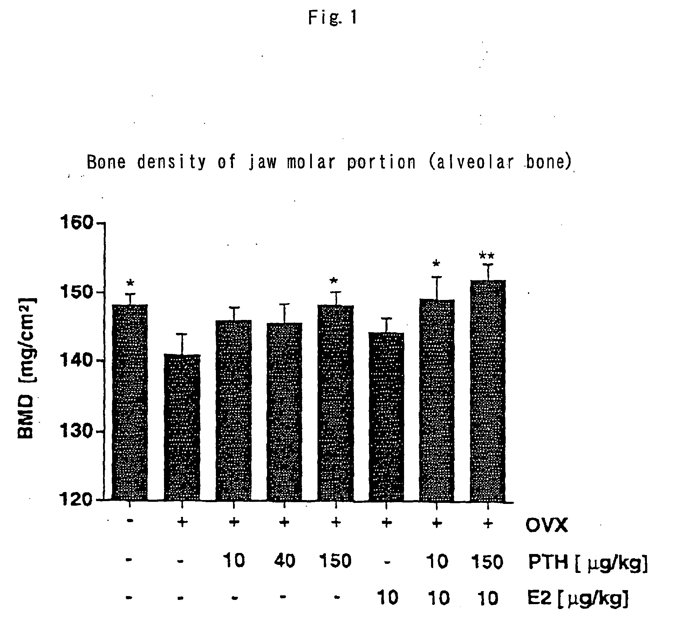

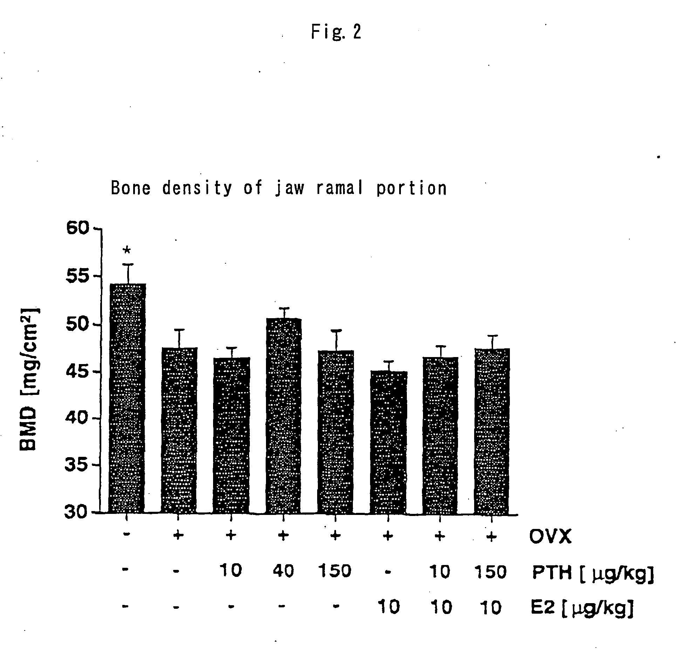

The results are shown in Figs. 1 and 2, in which PTH designates human PTH (1-84) and E2, 17β-estradiol. Symbol "+" in the row of OVX designates the application of OVX and "-" means that OVX was not performed.

According to these figures, OVX significantly lowered the bone densities of the femur and lumbar vertebrae in rats. Upon administration of human PTH (1-84), the bone densities of the femur and lumbar vertebrae in the ovariectomized rats increased in a dose-dependent manner and, at a dose of 150 µg/kg body weight, almost recovered to the level for the Sham operation group. The bone density of the mandibular alveolar bone portion decreased significantly upon OVX; the group administered 150 µg/kg body weight of human PTH (1-84) and the group administered both 17β-estradiol and 10 µg/kg body weight of human PTH (1-84) saw significant increases over the OVX group and returned to the level for the Sham operation group. The elevation in bone density was the most marked in the group administered both 17β-estradiol and 150 µg/kg body weight of human PTH (1-84). The bone density of the ramal portion also decreased significantly upon OVX but did not recover upon administration of 17β-estradiol or human PTH (1-84); obviously, the effect of OVX and the influence of the administration of the two hormones differed between the alveolar bone portion and the ramal portion.

When aged female rats were ovariectomized, the mandibular alveolar portion and the ramal portion showed the same decrease in bone density as was observed in the femur and lumbar vertebrae. When the ovariectomized rats were administered human PTH (1-84) having a bone formation promoting action and recently receiving attention as a therapeutic of osteoporosis, a distinct improvement in bone density occurred in the mandibular bone and its efficacy was enhanced by 17β-estradiol. Combined administration of human PTH (1-84) and 17β-estradiol exhibited outstanding efficacy in preventing the weakening of the mandibular bone and enhancing bone regeneration after placement of an implant.

### Example 2

### (Method)

Twenty-three female SD rats (body weight; 230 - 250 g) were used. At the age of 20 weeks after birth, 15 of the animals were subjected to OVX (ovariectomy) and the remaining eight were subjected to Sham operation. Then, at the age of 22 weeks after birth, ten of the ovariectomized rats and four in the Sham operation group had the posterior teeth in the upper jaw extracted. Starting at the age of 30 weeks when bone resorption progressed fully, five of the ovariectomized rats that had the posterior teeth in the upper jaw extracted were subcutaneously administered with human PTH (1-84) at a dose of 150 µg/kg body weight for 10 weeks on a four-time-a-week basis. After the administration of human PTH (1-84) and at the age of 40 weeks after birth, the whole mandibular bone was extracted and the bone densities and bone mineral contents in the whole mandibular bone and the mandibular molar portion were measured by DXA (Aloca).

### (Results)

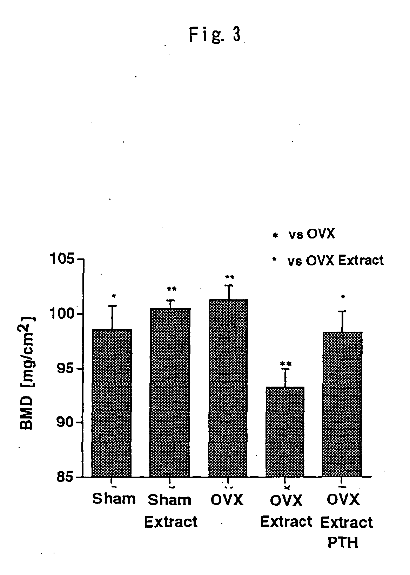

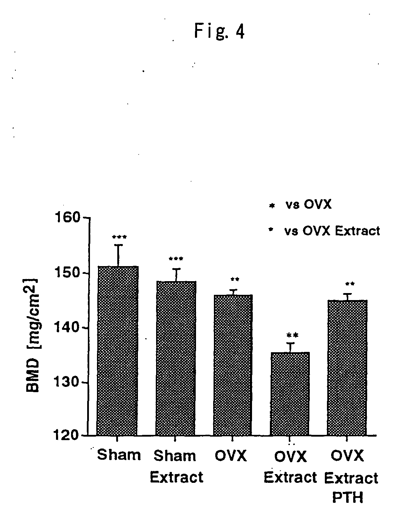

The results are shown in Figs. 3 and 4. Fig. 3 is a graph showing the differences in the bone density of the whole mandibular bone depending on whether OVX was practiced, whether tooth removal was effected and whether human PTH (1-84) was administered. Fig. 4 is a graph showing the differences in the bone density of the mandibular molar portion depending on whether OVX was practiced, whether tooth removal was effected and whether human PTH (1-84) was administered. In these figures, "Sham" designates the application of Sham operation at the age of 20 weeks after birth, "Sham, Extract" the application of Sham operation at the age of 20 weeks after birth and tooth removal at the age of 22 weeks after birth, "OVX" the application of ovariectomy at the age of 20 weeks after birth, "OVX Extract" the application of ovariectomy at the age of 20 weeks after birth and tooth removal at the age of 22 weeks after birth, and "OVX, Extract, PTH" the application of ovariectomy at the age of 20 weeks after birth and tooth removal at the age of 22 weeks after birth, followed by administration of human PTH (1-84) for 10 weeks starting at the age of 30 weeks after birth. BMD designates bone density. According to these figures, tooth removal effected after OVX performed caused marked drops in the bone mineral contents of whole mandibular bone and the mandibular molar portion; however, upon PTH administration, their bone mineral contents increased noticeably. These results show that from a clinical viewpoint, the efficacy of PTH is believed to be exhibited more noticeably in patients suffering from tooth loss, for example, patients, in particular, elderly ones who have lost antagonistic teeth or patients with edentulous jaws.

### INDUSTRIAL APPLICABILITY

As described above, (1) parathyroid hormone (PTH) or PTH derivatives or (2) PTH or PTH derivatives and estrogen have various actions such as increasing the bone density of the jaw alveolar bone portion, particularly the one in the upper jaw or the lower jaw, preventing the weakening of the jaw alveolar bone portion, particularly the one in the upper jaw or the lower jaw and promoting bone regeneration and, hence, are useful as orthodontic therapeutics. In addition, they have an action in increasing the bone densities of the whole mandibular bone and the mandibular molar portion after tooth removal; hence, clinically, more noticeable efficacy is anticipated in patients suffering from tooth loss, for example, patients in particular, elderly ones who have lost antagonistic teeth or patients with edentulous jaws.

## Claims

1. Use of (1) parathyroid hormone (PTH) or one or more PTH derivatives or (2) PTH or one or more PTH derivatives and estrogen for the preparation of a dental therapeutic.

2. The use according to claim 1, for the enhancement of the bone density of the jaw alveolar bone portion.

3. The use according to claim 1, for the prevention and therapy of the weakening of the jaw alveolar bone portion.

4. The use according to claim 1, for the promotion of the bone regeneration of the jaw alveolar bone portion.

5. The use according to claim 1, for the dental therapy after tooth removal or upon tooth loss.

6. The use according to any one of claims 1 - 5, wherein the parathyroid hormone (PTH) is human PTH (1-84).

7. The use according to claim 6, which contains substantially pure human PTH (1-84).

8. The use according to claim 6 or 7, wherein human PTH (1-84) is recombinant human PTH (1-84).

9. The use according to any one of claims 1 - 5, wherein the PTH derivative is human PTH (1-34).

10. The use according to claim 9, which contains substantially pure human PTH (1-34).

11. The use according to claim 9 or 10, wherein human. PTH (1-34) is recombinant human PTH (1-34).

12. The use according to any one of claims 1-8, comprising (1) parathyroid hormone (PTH) or (2) PTH and estrogen as an active ingredient or active ingredients.

13. The use according to any one of claims 1-5, and 9-11 comprising (1) a parathyroid hormone (PTH) derivative or (2) a PTH derivative and estrogen as an active ingredient or active ingredients.

14. Use according to any one of claims 1-13 for the preparation of a PTH non invasive preparation.
